# EUROPÄISCHE PATENTSCHRIFT

(11) **EP 0 989 407 B1**
(45) Veröffentlichungstag und Bekanntmachung des Hinweises auf die Patenterteilung: **26.02.2003**
(21) Anmeldenummer: 99118291.6
(22) Anmeldetag: 15.09.1999
(51) Int. Cl.: G01N 33/72, G01N 33/58, G01N 33/558

(54) **Verfahren zur Bestimmung von glykiertem Hämoglobin**
Method for the determination of glycosylated haemoglobin
Méthode pour la détermination de l'hémoglobine glycosylée

(30) Priorität: 21.09.1998 DE 19843094
(43) Veröffentlichungstag der Anmeldung: 29.03.2000
(73) Patentinhaber: Roche Diagnostics GmbH, 68298 Mannheim (DE)
(72) Erfinder: Pachl, Rudolf, Dr., 67158 Ellerstadt (DE); Horn, Carina, Dr., 68199 Mannheim (DE)

(56) Entgegenhaltungen:
- WO-A-90/13818
- WO-A-92/08722
- WO-A-96/03657
- DE-A- 3 720 736
- US-A- 4 861 728

## Beschreibung

Die Erfindung betrifft ein Verfahren zur Bestimmung des Verhältnisses von glykiertem zu nicht-glykiertem Hämoglobin in einer Probe. Weiterhin betrifft die Erfindung ein analytisches Testelement, das zur Bestimmung des Verhältnisses von glykiertem zu nichtglykiertem Hämoglobin in einer Probe anhand des neuen, erfindungsgemäßen Verfahrens geeignet ist.

Bei Patienten mit Diabetes mellitus ist die Glykierung von Hämoglobin und Serumproteinen erhöht. Die Erhöhung ist abhängig von der Glukosekonzentration und der Inkubationszeit des Proteins mit Glukose. Die Glykierung von Serumproteinen, einschließlich Hämoglobin erfolgt in diesen Fällen nicht enzymatisch, sondern unkatalysiert durch chemische Reaktion der Glukose mit Aminogruppen der Proteine. Die Fachwelt geht davon aus, daß die Konzentration eines bestimmten Protein-Glukose-Addukts sowohl die Glukosekonzentration über eine bestimmte Zeit als auch die Umsatzrate des Proteins widerspiegelt. Glykiertes Hämoglobin wird als Indikator der mittleren Blutglukosekonzentration während der letzten zwei bis drei Monate vor Blutabnahme und Untersuchung angesehen. Glykiertes Serumprotein gibt die Blutglukosekonzentration während einer kürzeren Zeitspanne wieder. Die Bestimmung von glykiertem Protein, wie glykiertem Hämoglobin (insbesondere HbA1c) oder glykiertem Serumprotein hat deshalb eine erhebliche Bedeutung für die langfristige glykämische Kontrolle von Diabetespatienten. Insbesondere ist in diesem Zusammenhang das Verhältnis von glykiertem Hämoglobin zu Gesamthämoglobin in einer Blutprobe aussagekräftig.

Der Gehalt an und die Bestimmung von Hämoglobin A1 (HbAlc) bzw. dem dazu medizinisch äquivalenten glykierten Hämoglobin ist ein wichtiges medizinisches Kriterium bei der langfristigen Stoffwechseleinstellung von diabetischen Patienten. Bei ca. 80 % des glykierten Hämoglobins befindet sich die Glucose an dem N-terminalen Valin der β-Kette (sogenanntes "HbAlc"), während sich die Kohlenhydratreste des restlichen glykierten Hämoglobins an anderen Stellen befinden, bzw. andere Zucker außer Glukose mit Hämoglobin reagiert haben.

Zur Bestimmung von glykiertem Hämoglobin in Blut sind zumindest 5 Prinzipien derzeit gebräuchlich. Diese sollen im folgenden kurz beschrieben werden:

### 1. Ionenaustauschchromatographie mit einer Mikrosäule

Die Ionenaustauschchromatographie mit Hilfe einer Mikrosäule wird beispielsweise beschrieben in J. Clin. Chem. Clin. Biochem., 19, 1097-1101 (1981), Lab. Med., 8, 74-78 (1984) und J. Am. Chem. Soc., 83, 1472-1478 (1961). Bei diesem Verfahren wird das Hämoglobin einer hämolysierten Probe an einen Ionenaustauscher gebunden und fraktioniert eluiert. Durch einen Eliminator im Hämolysereagenz wird die labile Aldiminform des HbA1 abgebaut. Die Trennung von nicht-glykiertem Hämoglobin (HbA0) und des glykiertem Hämoglobins (HbA1) erfolgt aufgrund der unterschiedlichen Ladung der beiden Hämoglobinarten. Die Messung der Anteile an glykiertem und nicht-glykiertem Hämoglobin erfolgt mit einem Photometer bei einer Wellenlänge von 415 nm. Bei diesem Verfahren kann als Probe EDTA-Blut und Heparin-Blut verwendet werden.

Nachteilig an der Bestimmung von glykiertem Hämoglobin durch Ionenaustauschchromatographie mit einer Mikrosäule ist die große Temperatur- und pH-Wert Abhängigkeit sowie Interferenzen durch labile Vorläufer des glykierten Hämoglobins, Nicht-Glukose-Addukte des Hämoglobins und andere Hämoglobinvarianten. Zudem ist diese Methode nur in einem Analysenlabor durch geschultes Personal durchführbar.

### 2. Elektrophoretisches Verfahren

Das Elektrophoreseverfahren zur Bestimmung von glykiertem Hämoglobin wird beispielsweise in Clin. Chem., 34, 145-148 (1988) und Clin. Chem., 26, 1598-1602 (1980) beschrieben. Das Verfahren trennt HbA1 und HbA0 durch die Wirkung der Endoosmose aufgrund unterschiedlicher Ladungen der glykierten bzw. nicht-glykierten Hämoglobine. Der Elektrophoresefilm wird mit einem Densitometer ausgewertet. Als Probenmaterialien können EDTA-Blut und Oxalat-Blut eingesetzt werden. Hepannisiertes Frischblut kann fiir dieses Verfahren nicht verwendet werden.

Wie bei der Ionenaustauschchromatographie mit einer Mikrosäule interferieren beim elektrophoretischen Verfahren labile Vorläufer des glykierten Hämoglobins, Nicht-Glukoseaddukte und anderer Hämoglobinvarianten. Auch das elektrophoretische Verfahren ist nur in einem Analyselabor von geschultem Personal durchzuführen.

### 3. Affinitätschromatographie

Die Bestimmung von glykiertem Hämoglobin mittels Affinitätschromatographie beruht auf dem Prinzip der Trennung von glykiertem und nicht-glykiertem Hämoglobin auf einer Agarosesäule, die kovalent gebundene Aminophenylboronsäure enthält. Das Verfahren ist beschrieben beispielsweise in Diabetes, 33, 73-76 (1984), Fresenius Z. Anal. Chem., 317, 703-704 (1984), Diabetologia, 27, 56-58 (1984) sowie in Clin. Chim. Acta. 168, 81-86 (1987).

Das auf der Agarosesäule immobilisierte Trihydroxyaminophenylboran bildet mit der Ketoaminform des glykierten Hämoglobins einen Komplex, so daß diese Fraktion auf der Säule zurückgehalten wird. Das nicht-glykierte Hämoglobin passiert ohne Verzögerung als Hauptfraktion die Säule und wird aufgefangen. Das glykierte Hämoglobin wird mit sorbithaltigem Puffer von der Säule eluiert und ebenfalls separat aufgefangen. Aus den Extinktionen der Fraktionen errechnet man den Anteil des glykierten Hämoglobins.

Die affinitätschromatographische Trennung von glykiertem und nicht-glykiertem Hämoglobin zeichnet sich dadurch aus, daß kaum pH-Wert- und Temperatureinflüsse zu verzeichnen sind und keine Interferenzen durch die labile Aldiminform, andere Hämoglobinvarianten sowie Nicht-Glukoseaddukte von Hämoglobin existieren. Als Probenmaterial eignet sich das Hämolysat von Fluorid- und EDTA-Blut. Die Methode beinhaltet mehrere Durchführungsschritte und ist nur in einem Analyselabor von geschultem Personal durchführbar.

### 4 Immunologische Bestimmung

Die immunologische Bestimmung von glykiertem Hämoglobin ist beispielsweise in Klin. Lab., 39, 991-996 (1993) beschrieben. Bei diesem Verfahren reagiert das glykierte Hämoglobin (HbAlc) der Probe in einem ersten Reaktionsschritt mit Anti-HbAlc-Antikörpem. Da das HbAlc-spezifische Epitop in jeder β-Kette des glykiertem Hämoglobins nur einmal vorkommt, kommt es zu keiner Aggregation des Antigen-Antikörperkomplexes. Erst nach Zugabe eines Polyhaptens, das mehrere HbAlc-spezifische Epitope hat, reagieren die Moleküle mit den überschüssigen Antikörpern aus der ersten Reaktion und es kommt zur Bildung eines unlöslichen Immunkomplexes. Dieser kann turbidimetrisch gemessen werden. Das turbidimetrische Signal ist umgekehrt proportional zur Konzentration des glykierten Hämoglobins in der Probe.

Nachteilig an diesem Verfahren, das mehrere Durchführungsschritte beinhaltet, ist, daß es nur in einem spezialisierten Analysenlabor durchgerührt werden kann.

### 5. Nachweis des glykierten Hämoglobins mit Hilfe von Boronsäure-Farbstoff-Konjugaten

Der Nachweis des glykierten Hämoglobins mit Hilfe von Boronsäure-Farbstoff-Konjugaten wird beispielsweise in EP-B 0 471 774, EP-B 0 557 357 sowie in US 5,506,144 und US 5,631,364 beschrieben. Bei diesem Verfahren bindet nach der Lyse der Erythrozyten ein Konjugat aus Phenylboronsäure und einem blauen Farbstoff an die glykierten Stellen des glykierten Hämoglobins. Dazu wird nach der Hämolyse der Blutprobe eine Reagenzienmischung, die das Boronsäure-Farbstoff-Konjugat enthält, zum Hämolysat gegeben, die Gesamtmenge des Hämoglobins gefällt und durch Filtration vom Rest der Probe getrennt. Das Boronsäure-Farbstoff-Konjugat ist dabei an den Zuckerrest des glykierten Hämoglobins gebunden. Mit Hilfe einer Waschlösung wird nicht-gebundener Farbstoff anschließend, entfernt und das Filtrationsmedium bei den Wellenlängen 470 nm (für die Gesamtmenge an Hämoglobin) und 630 nm (für den blauen Farbstoff, der über die Boronsäure an das glykierte Hämoglobin gebunden ist), vermessen.

Nachteilig an diesem Verfahren ist, daß mehrere Schritte zur Durchführung erforderlich sind, bei denen jeweils die benötigten Flüssigkeiten (Probe, Fällungslösung, Waschlösung) genau dosiert werden müssen.

DE-A 37 20 736 offenbart ein Verfahren zum Nachweis von glykierten Serumproteinen in biologischen Proben. Hierzu wird eine fluoreszierende Arylboronsäure zu einer Probe gegeben und die überschüssige, nicht an Serumproteine gebundene Boronsäure von der Probe abgetrennt. Anschliessend wird der Gehalt an glykiertem Albumin bestimmt. Die Gegenwart von Hämoglobin stört bei diesem Verfahen den Nachweis von glykiertem Albumin, da glykiertes Hämoglobin mitbestimt wird.

Aufgabe der Erfindung ist es, die Nachteile des Standes der Technik zu beseitigen. Insbesondere ist es Aufgabe der vorliegenden Erfindung, ein einfaches und schnelles Verfahren und ein entsprechendes analytisches Testelement zur Bestimmung des Verhältnisses von glykiertem zu nichtglykiertem Hämoglobin in einer Probe zur Verfügung zu stellen.

Die Aufgabe wird durch den Gegenstand der Erfindung, wie er in den Patentansprüchen definiert ist, gelöst.

Gegenstand der Erfindung ist ein Verfahren zur Bestimmung des Verhältnisses von glykiertem zu nicht-glykiertem Hämoglobin in einer Probe, wobei man
(i) gegebenenfalls die Probe hämolysiert, um zellgebundenes Hämoglobin freizusetzen,
(ii) die Probe mit einem Überschuß an signalbildenden Molekülen in Kontakt bringt, wobei ein Teil der signalbildenden Moleküle an glykiertes Hämoglobin in der Probe bindet,
(iii) den nicht an glykiertes Hämoglobin gebundenen Teil der signalbildenden Moleküle von der Probe abtrennt,
(iv) in der Probe, die an glykiertes Hämoglobin gebundene signalbildende Moleküle sowie nicht-glykiertes Hämoglobin enthält, die signalbildenden Moleküle als Maß fiir das glykierte Hämoglobin und die Gesamtmenge an Hämoglobin bestimmt, und
(v) aus den gemäß Schritt (iv) erhaltenen Werten das Verhältnis von glykiertem zu nichtglykiertem Hämoglobin in der Probe bestimmt.

Das erfindungsgemäße Verfahren eignet sich zur Bestimmung des relativen Anteils an glykiertem Hämoglobin bezogen auf die Gesamtmenge Hämoglobin in einer Blutprobe. Als glykiertes Hämoglobin (GHb), welches bisweilen auch als glykosyliertes bzw. glykosidiertes Hämoglobin bezeichnet wird, ist diejenige Hämoglobinfraktion zu verstehen, welche die Hauptfraktionen Hämoglobin Ala (HbAla), Hämoglobin Alb (HbAlb) und Hämoglobin Alc (HbAlc) enthält.

Das erfindungsgemäße Verfahren eignet sich insbesondere für Blut als Probenmaterial. Dabei kann frisches Kapillarblut ebenso verwendet werden wie mit Antikoagulantien behandeltes Blut oder eingetrocknetes und wieder aufgelöstes Blut, wie es beispielsweise bei der Verwendung sogenannter Probenbriefchen anfällt. Als Probenmaterial eignet sich auch hämolysiertes Blut (Hämolysat) oder aus Blut isoliertes Hämoglobin, welches gegebenenfalls zur Analyse mit dem erfindungsgemäßen Verfahren wieder gelöst werden muß.

Durch die Hämolyse der Blutprobe wird zellgebundenes Hämoglobin, beispielsweise aus Erythrozyten, freigesetzt und so für die erfindungsgemaße Bestimmung erst zugänglich gemacht. Die Hämolyse der Blutprobe kann durch an sich bekannte Verfahren bewirkt werden, wie z. B. durch Zugabe von Hämolysereagenzien wie Saponin, Natriumdodecylsulfat (SDS) und/oder quartären Ammoniumsalzen, wobei die Zugabe von Saponin bevorzugt ist.

Für das erfindungsgemäße Verfahren ist es von Bedeutung, daß das glykierte Hämoglobin in der Probe mit signalbildenden Molekülen in Kontakt gebracht wird, die selektiv an das glykierte Hämoglobin binden und dieses dadurch markieren und detektierbar machen. Als signalbildende Moleküle eignen sich insbesondere Dirketlabel, Enzymlabel oder Radiolabel, die über eine Boronsäuregruppe oder andere spezifische Bindungspartner, wie z. B. Antikörper, selektiv an glykiertes Hämoglobin binden können, nicht jedoch an nicht-glykiertes Hämoglobin.

Vorzugsweise sind die signalbildenden Moleküle ein Konjugat aus einem oder mehreren Dihydroxyborylresten oder Salzen davon, die an eine signalbildende Markierung gebunden sind. Ganz besonders bevorzugt sind dies Boronsäurekonjugatverbindungen oder deren Salze mit einem Absorptionsmaximum, das bei einer Wellenlänge liegt, die außerhalb des Bereichs liegt, in dem Hämoglobin absorbiert. Vorzugsweise sollte das Absorptionsmaximum über 600 nm liegen. Diese Verbindungen können durch die allgemeine Formel I beschrieben werden,

**V-W-B(OH)**_{**2**} (I)

in der V ein Cyanin-, Phenoxazin-, Phenothiazin- oder Triphenylmethanchromophor- und/oder -fluorophorrest ist, der ein Absorptionsmaximum bei nicht weniger als 600 nm aufweist, und W eine organische Brückengruppe ist. Derartige Verbindungen an sich und deren Verwendung in Bestimmungsverfahren für glykierte Proteine sind dem Fachmann in einer großen Vielzahl bekannt, beispielsweise aus US 5,631,364, EP-A 0 557 357 und dem darin zitierten Stand der Technik. Auf diese Dokumente wird hier ausdrücklich Bezug genommen.

Das Inkontaktbringen der Probe mit den signalbildenden Molekülen kann dadurch geschehen, daß die signalbildenden Moleküle als Feststoff, Lösung, Dispersion etc. zur Probe gegeben werden. Alternativ ist es auch möglich, die Probe zu den signalbildenden Molekülen, die beispielsweise löslich in einem Reaktionsgefäß oder einer porösen Matrix enthalten sind, zu geben. Wichtig ist dabei, daß die signalbildenden Moleküle in der Probe gelöst werden können. Sie dürfen deshalb nicht unlösbar immobilisiert vorliegen.

Um sicherzustellen, daß ein möglichst großer Anteil des glykierten Hämoglobins in der Probe mit den signalbildenden Moleküle in Wechselwirkung tritt, ist es vorteilhaft, einen Überschuß an signalbildenden Molekülen einzusetzen. Ein Teil der signalbildenden Moleküle bindet bei der Durchführung des erfindungsgemäßen Verfahrens an glykiertes Hämoglobin. Vorzugsweise wird sämtliches in der Probe enthaltenes glykiertes Hämoglobin durch signalbildende Moleküle gebunden und so detektierbar und von nicht-glykiertem Hämoglobin unterscheidbar gemacht. Die Bindung der signalbildenden Moleküle an das glykierte Hämoglobin kann, wie oben erwähnt, beispielsweise durch selektive, affine Wechselwirkungen eines Molekülteils der signalbildenden Moleküle mit dem glykierten Hämoglobin bewirkt werden. Beispielsweise kann dies durch eine Antigen-Antikörper-Wechselwirkung oder durch die erfindungsgemäß bevorzugte Affinität von Boronsäuren zu Diolstrukturen im Zuckerrest des glykierten Hämoglobins geschehen.

Für das erfindungsgemäße Verfahren ist es wesentlich, daß der Teil der signalbildenden Moleküle, der nicht durch Inkontaktbringen mit der Probe an glykiertes Hämoglobin gebunden ist, von der Probe abgetrennt wird. Hierin unterscheidet sich das erfindungsgemäße Verfahren wesentlich von den Verfahren des Standes der Technik. Im Stand der Technik wird jeweils Hämoglobin, glykiertes Hämoglobin und markiertes glykiertes Hämoglobin gemeinsam oder fraktioniert von der Probe getrennt und nach dem Trennen von der Probe separat oder gemeinsam bestimmt. Das erfindungsgemäße Verfahren verzichtet auf die Abtrennung der Hämoglobine von der Probe. Dadurch entfallen die aufwendigen Abtrennschritte, die in den bisher üblichen Verfahren notwendig waren und zu nur schwer automatisierbaren, mehrstufigen und langsamen Bestimmungsverfahren geführt haben.

Für die Abtrennung der signalbildenden Moleküle, die nach Inkontaktbringen der Probe mit ihnen in dieser gelöst vorliegen, von der Probe kommen erfindungsgemäß mehrere Möglichkeiten in Betracht. Vorzugsweise wird hierfür ausgenutzt, daß die signalbildenden Moleküle einen Rest besitzen, der selektiv mit glykiertem Hämoglobin in Wechselwirkung treten kann. Diese selektive Wechselwirkung kann - wie oben erwähnt - beispielsweise darin bestehen, daß die signalbildenden Moleküle einen Antikörper gegen glykiertes Hämoglobin tragen oder einen Boronsäurerest aufweisen, der sich mit Diolstrukturen des glykierten Hämoglobins verbinden kann. Das Abtrennen der signalbildenden Moleküle aus der Probe wird in einer Ausführungsform des erfindungsgemäßen Verfahrens dadurch erreicht, daß der nicht an glykiertes Hämoglobin gebundene Anteil der signalbildenden Moleküle durch Inkontaktbringen mit immobilisiertem glykiertem Hämoglobin (für den Fall, daß Antikörper dagegen eingesetzt werden) oder mit immobilisierten Diolen (für den Fall, daß Boronsäurereste eingesetzt werden) an diese Strukturen gebunden wird und somit aus der Probe entfernt wird. Dies kann beispielsweise dadurch geschehen, daß die Probe enthaltend nicht-gebundene signalbildende Moleküle ein durchströmungsfähiges Material, welches die oben genannten immobilisierten Strukturen enthält, durchströmt. Es ist jedoch auch möglich, die oben genannten immobilisierten Strukturen auf magnetischen Partikeln zu immobilisieren und nach Inkubation der Probe mit den Magnetpartikeln und erfolgter Bindung der signalbildenden Moleküle an die Magnetpartikel diese durch anlegen eines Magnetfeldes aus der Probe zu entfernen. Bei all diesen Varianten ist es vorteilhaft, daß die Affinität der signalbildenden Moleküle zu glykiertem Hämoglobin in der Probe höher ist als zu den immobilisierten Strukturen, so daß zuvor gebildete Komplexe aus glykiertem Hämoglobin aus der Probe mit den signalbildenden Molekülen für die nachträgliche Detektion erhalten bleiben.

Erfindungsgemäß ist es bevorzugt, die signalbildenden Moleküle, vorzugsweise die oben beschriebenen Boronsäure-Farbstoff-Konjugate, durch Ausnutzen elektrostatischer Wechselwirkungen von der Probe abzutrennen. Hierbei macht man sich Zunutze, daß bei pH-Werten, die mindestens um eine Einheit größer sind als der pKₛ der Boronsäure-Farbstoff-Konjugate, die freien, das heißt nicht an glykiertes Hämoglobin gebundenen Boronsäure-Farbstoff-Konjugate, eine im Vergleich zum freien Hämoglobin sowie zum Komplex aus glykiertem Hämoglobin und Boronsäure-Farbstoff-Konjugat höhere negative Ladungsdichte aufweisen. Das Abtrennen der freien Boronsäure-Farbstoff-Konjugate von der Probe kann in diesem Fall beispielsweise dadurch erzielt werden, daß die Probe durch ein poröses, durchströmungsfähiges Material geleitet wird, welches nach außen hin positiv geladen ist. Beispielsweise hat sich hierfür Membranmaterial aus Nylon, insbesondere ein mit quartären Ammoniumgruppen modifiziertes Nylon 66™, welches beispielsweise als Biodyne B™ von der Fa. Pall vertrieben wird, als besonders geeignet erwiesen. Während die Probe und insbesondere das darin enthaltene Hämoglobin sowie der Komplex aus glykiertem Hämoglobin und Boronsäure-Farbstoff-Konjugat schnell in und durch das poröse, durchströmungsfähige Material strömen kann, ist dies für die freien Boronsäure-Farbstoff-Konjugate nicht möglich. Die Bestimmung von (Gesamt-)Hämoglobin und glykiertem Hämoglobin kann dabei in dem druchströmungsfähigen Material oder nach dem Durchströmen der Probe durch das durchströmungsfähige Material erfolgen.

Ebenfalls ist es möglich, für die Trennung von Hämoglobin und markierten glykiertem Hämoglobin von den signalbildenden Molekülen andere molekulare Wechselwirkungen, beispielsweise Wechselwirkungen mit adsorptionsfähigen Materialien, wie z. B. Kieselgel und Aluminiumoxid, auszunutzen.

Für die Bestimmung von Hämoglobin und glykiertem Hämoglobin kann im Prinzip auf bekannte Verfahren zurückgegriffen werden. Insbesondere bevorzugt ist für Hämoglobin die Messung von Absorption, Reflektion oder Fluoreszenz. Diese Messverfahren sind für glykiertes Hämoglobin analog anwendbar, wobei im erfindungsgemäßen Verfahren hier vorzugsweise bei Wellenlängen gemessen wird, bei denen Hämoglobin selbst keine Absorption, Reflektion bzw. Fluoreszenz zeigt. Dies ergibt sich aus der Tatsache, daß bei dem erfindungsgemäßen Verfahren keine Separation von nicht-glykiertem Hämoglobin und glykiertem Hämoglobin stattfindet und somit beide Hämoglobine nebeneinander vorliegen und detektiert werden müssen. Für die Bestimmung des glykierten Hämoglobins kommen je nach Art des signalbildenden Moleküls noch die Bestimmung der Enzymaktivität (bei Enzymlabel) oder der Radioaktivität (bei Radiolabel) hinzu.

Die Bestimmung des Verhältnisses von glykiertem zu nicht-glykiertem Hämoglobin in der Probe bzw. die dazu äquivalente Bestimmung des prozentualen Anteils von glykiertem Hämoglobin am Gesamthämoglobingehalt der Probe erfolgt ebenfalls nach an sich üblichen, dem Fachmann bekannten Verfahren. Vorzugsweise erfolgt die Verhältnisbestimmung über Eichkurven mit bekannten Hb/GHb-Werten und Vergleich der Meßdaten mit den Eichdaten.

Ein weiterer Gegenstand der Erfindung ist ein analytisches Testelement. das zur Bestimmung des Verhältnisses von glykiertem zu nichtglykiertem Hämoglobin in einer Probe, insbesondere anhand des neuen, erfindungsgemäßen Verfahrens, geeignet ist. Das erfindungsgemäße Testelement enthält neben- und/oder übereinander angeordnet
- eine Probenaufgabezone,
- eine Reagenzienzone, in der Hämolysereagenzien zur Freisetzung von zellgebundenem Hämoglobin vorliegen und/oder die signalbildende Moleküle nicht-immobilisiert enthält,
- eine Abtrennzone zum Abtrennen von nicht an glykiertem Hämoglobin gebundenen signalbildenden Molekülen, sowie
- eine Nachweiszone, wobei
die Zonen auf oder in einem oder mehreren porösen Matrixmaterialien untergebracht sind und untereinander in einem Flüssigkeitsübertritt ermöglichendem Kontakt stehen oder in einen solchen gebracht werden können.

Die einzelnen Zonen des erfindungsgemäßen Testelements bezeichnen funktionale Bezirke innerhalb der Testelementarchitektur. Sie können sowohl als flächige, d. h. im wesentlichen zweidimensionale, als auch als dreidimensionale Bezirke ausgebildet sein. Es ist erfindungsgemäß möglich, daß ein physikalischer Bezirk, bspw. ein Abschnitt eines porösen Matrixmaterials, des Testelements mehrere Zonen in sich vereint. Beispielsweise kann die Probe direkt auf einen reagenzienhaltigen Bezirk des Testelements aufgegeben werden. In diesem Fall befinden sich Probenaufgabezone und Reagenzienzone in ein und demselben physikalischen Bezirk. Ebenso ist es möglich, daß Nachweiszone und Abtrennzone in einem gemeinsamen physikalischen Bezirk vorliegen.

Erfindungsgemäß liegen die Zonen auf oder in einem oder mehreren porösen Matrixmaterialien, wobei es bevorzugt ist, daß das Testelement zumindest zwei Matrixmaterialien enthält. Als poröse Matrixmaterialien sind beispielsweise Vliese, Papiere, Gewebe und Gewirke aus natürlichen oder synthetischen organischen oder anorganischen faserförmigen Materialien geeignet. Ebenfalls geeignet sind Membranen, Schwämme, Dochte, Schaumstoffe, Adsorbentien wie Kieselgel oder Aluminiumoxid enthaltende Schichten und dergleichen mehr. Bevorzugt sind Vliese, Gewebe und Membranen. Der Flüssigkeitstransport in dem oder den Matrixmaterialien beruht im wesentlichen auf hydrostatischen und/oder kapillaren Kräften.

Die einzelnen Zonen sollen erfindungsgemäß zueinander in einem flüssigkeitsübertrittermöglichendem Kontakt stehen oder in einen solchen gebracht werden können. Probenflüssigkeit, die auf die Probenaufgabezone aufgegeben wird, muß von dort in die Reagenzienzone eindringen können. Von dort muß die Probenflüssigkeit die Abtrennzone erreichen können und nach dem Abtrennen der nicht an glykiertem Hämoglobin gebundenen signalbildenden Moleküle in die Nachweiszone vordringen können. Vorzugsweise wird dies dadurch erreicht, daß die einzelnen Zonen, die zumindest teilweise unterschiedliche Matrixmaterialien aufweisen können, sich jeweils paarweise berühren oder gegebenenfalls sogar ganz oder teilweise überlappen. Ebenfalls möglich ist es, daß die einzelnen Zonen auf oder in einem einzigen Matrixmaterial enthalten sind. Es ist jedoch andererseits auch möglich, daß zwischen den einzelnen, bisher genannten Zonen weitere Zonen vorhanden sind, die jedoch den Flüssigkeitsübertritt von einer Zone zur nächsten ermöglichen. Solche Zonen können generell als Transportzonen bezeichnet werden. Ebenso ist es möglich, daß einzelne Zonen zunächst nicht in einem flüssigkeitsübertrittermöglichenden Kontakt zueinander stehen, sondern erst durch äußeres Einwirken in einen solchen gebracht werden. Beispielsweise können die Abtrenn- und Nachweiszone gemeinsam auf einem separaten Träger untergebracht sein, der mit dem Träger, auf dem die Probenaufgabe- und die Reagenzienzonen befestigt sind, über eine bewegliche Verbindung, z. B. ein Scharnier, verbunden ist. Dieser Träger stellt dann quasi eine Klappe dar, die durch Aufdrücken der die Zonen enthaltenden Flächen des einen Trägers auf den zweiten Träger einen flüssigkeitsübertrittermöglichenden Kontakt zwischen Reagenzienzone und Abtrennzone herstellt. Ähnliche Konstruktionen sind prinzipiell bekannt und beispielsweise in US 5,426,030 beschrieben.

Die einzelnen Zonen können entweder im wesentlichen nebeneinander oder im wesentlichen übereinander angeordnet sein. Es ist auch möglich, daß ein Teil der Zonen im wesentlichen übereinander angeordnet ist, während ein anderer Teil der Zonen im wesentlichen nebeneinander angeordnet ist.

Das oder die porösen Matrixmaterialien können entweder selbsttragend sein oder auf bzw. in ein tragfähiges, nichtsaugfähiges Material aufgebracht oder eingebracht sein. Vorzugsweise sind die erfindungsgemäß geeigneten Matrixmaterialien nicht selbsttragend. Bevorzugt sind diese deshalb auf ein tragfähiges Material als inertem Träger aufgebracht. Als tragfähige Materialien eignen sich z. B. Kunststoffolien und -formteile, beschichtete Pappen, Glas, Keramik, Metallbleche und dergleichen mehr. Vorzugsweise sollten die Träger inert gegenüber den verwendeten Probenmaterialien und Reagenzien sein, nicht von diesen angegriffen werden oder mit ihnen reagieren. Als erfindungsgemäß geeignet haben sich beispielsweise Folien aus inerten, wasserbeständigen Kunststoffen wie Polyethylen, Polypropylen, Polystyrol, Polycarbonat, Polyethylenterephthalat und dergleichen mehr herausgestellt.

Bei der Verwendung von Folien oder anderen schichtförmigen Materialien als inerten Trägern werden die porösen Matrixmaterialien auf diesen befestigt, beispielsweise durch Kleben oder Schweißen. Vorzugsweise werden die Matrixmaterialien durch doppelseitige Klebebänder oder durch Schmelzkleber auf den Trägern befestigt. Werden dreidimensionale Kunststofformteile als Träger - oder in diesem Fall besser als Gehäuse bezeichnet- verwendet, ist es beispielsweise auch möglich, die Matrixmaterialien zwischen zwei Gehäusehälften, die zu einem Gehäuse geschlossen werden können, einzuklemmen. Derartige und weitere Monatgetechniken sind dem Fachmann bekannt.

Die Probenaufgabezone des erfindungsgemäßen Testelements dient der Aufgabe der Probenflüssigkeit. Sie kann als eigener separater Bereich des Testelements ausgebildet sein oder aber Bestandteil der Reagenzienzone sein. Vorzugsweise ist die Probenaufgabezone für den Benutzer als solche gekennzeichnet, beispielsweise indem sie mit einem Zeichen versehen oder farblich gekennzeichnet ist. Die Probenaufgabezone grenzt direkt oder indirekt an die Reagenzienzone an und steht mit dieser in einem flüssigkeitsübertrittermöglichendem Kontakt, so daß Probenflüssigkeit von der Probenaufgabezone in die Reagenzienzone übertreten kann. Vorzugsweise ist die Probenaufgabezone eine der freiliegenden Oberflächen der Reagenzienzone bzw. des die Reagenzienzone enthaltenden porösen Matrixmaterials.

Die Reagenzienzone dient dazu, fiir den Nachweis von glykiertem Hämoglobin benötigte Reagenzien bereitzuhalten und die Wechselwirkung zwischen glykiertem Hämoglobin und den Reagenzien zu ermöglichen. Vorzugsweise enthält die Reagenzienzone die - bereits weiter oben im Zusammenhang mit dem erfindungsgemäßen Verfahren beschriebenen - signalbildenden Moleküle, die sich mit dem glykierten Hämoglobin zu einem detektierbaren Komplex verbinden können. Erfindungsgemäß liegen die signalbildenden Moleküle bezogen auf die zu erwartende Menge an glykiertem Hämoglobin der Probe im Überschuß vor. Weiterhin kann die Reagenzienzone Puffersubstanzen und weitere Hilfsstoffe, beispielsweise Hämolysereagenzien wie Saponin, Natriumdodecylsulfat (SDS) oder quartäre Ammoniumsalze enthalten. Die Reagenzien liegen vorzugsweise löslich als Feststoffe in der Reagenzienzone vor. Sie können untereinander gemischt und homogen verteilt in der Reagenzienzone enthalten sein. Es ist jedoch auch möglich, einzelne Kompartimente der Reagenzienzone mit unterschiedlichen Reagenzien auszustatten und diese gegebenenfalls sogar in unterschiedlichen porösen Matrixmaterialien unterzubringen, welche im Testelement zu einer Zone zusammengefaßt sein können. Die Reagenzien können nach an sich üblichen, dem Fachmann bekannten Verfahren, beispielsweise Imprägnieren der Reagenzien aus entsprechenden Lösungen, in die Reagenzienzone eingebracht werden. Vorzugsweise ist die Reagenzienzone ein poröses, inertes Vliesmaterial, beispielsweise ein Glasfaservlies oder ein Vlies aus Kunstfasern, in dem die Reagenzien homogen verteilt durch Imprägnieren und anschließendes Trocknen eingebracht wurden.

Erfindungsgemaß ist es auch möglich, nur einen Teil der Reagenzien, die zum Nachweis des glykierten Hämoglobins erforderlich sind, in der Reagenzienzone des erfindungsgemäßen Testelements bereitzustellen. Beispielsweise können in der Reagenzienzone nur Hämolysereagenzien vorliegen. Die signalbildenden Moleküle können dann entweder vor oder nach der Aufgabe der Probenflüssigkeit auf das Testelement zugegeben werden oder aber zusammen mit der Probe appliziert werden, nachdem diese gegebenenfalls mit den Reagenzien vermischt wurde. Ebenso ist es möglich, nur die signalbildenden Moleküle in der Reagenzienzone bereitzustellen und die anderen reagenzien, wie z. B. die Hämolysereagenzien, separat oder mit der Probenflüssigkeit zusammen aufzugeben.

In einer besonders bevorzugten Ausführungsform des erfindungsgemäßen Testelements enthält die Reagenzienzone ein mit signalbildenden Molekülen und gegebenenfalls Puffersubstanzen und weiteren Hilfsstoffen imprägniertes, poröses, inertes Vliesmaterial, auf welches auf der der Probenaufgabeseite zugewandten Seite ein Gewebe angebracht ist, welches Hämolysereagenzien enthält. In dieser bevorzugten Ausführungsform wird die Probe auf das Gewebe aufgegeben, welches mit dem Vlies in einem flüssigkeitsübertrittermöglichenden Kontakt steht.

Die Reagenzienzone des erfindungsgemäßen Testelements grenzt direkt oder indirekt an die Abtrennzone an. Beide Zonen stehen miteinander in einem flüssigkeitsübertrittermöglichendem Kontakt, so daß Probenflüssigkeit, die sich in der Reagenzienzone befindet, nach Inkubation mit den Reagenzien in die Abtrennzone übertreten kann.

Es hat sich als bevorzugt erwiesen, die Inkubationszeit der Probenflüssigkeit mit den Reagenzien der Reagenzienzone möglichst so lange zu gestalten, daß eine möglichst vollständige Komplexbildung zwischen signalbildenden Molekülen und glykiertem Hämoglobin stattfinden kann. Zu diesem Zweck ist es erfindungsgemäß bevorzugt, zwischen Reagenzienzone und Abtrennzone des Testelements eine Verzögerungszone einzubringen. Dabei ist zu berücksichtigen, ob das Lösen der Reagenzien in der Reagenzienzone oder die Reaktion der gelösten Reagenzien mit dem glykierten Hämoglobin geschwindigkeitsbestimmend sind. Je nachdem, welche Art von Geschwindigkeitsbestimmung vorliegt, eignen sich unterschiedliche Prinzipien bevorzugt fiir die Verzögerung.

Ist der geschwindigkeitsbestimmende Schritt hauptsächlich durch das Lösen der Reagenzien in der Probe gegeben, so ist die Probenflüssigkeit möglichst lange, d. h. möglichst bis zum vollständigen Lösen der Reagenzien, in der Reagenzienzone zu halten. Die Verzögerungszone kann in diesem Fall dadurch realisiert sein, daß ein verzögert lösliches Material, oder ein hydrophobierendes Material als Beschichtung an der Berührungs- bzw. Überlappungszone zwischen Reagenzienzone und Abtrennzone eingebracht wird. Probenflüssigkeit kann in diesem Fall nur verlangsamt in die Abtrennzone vordringen und wird so, verglichen mit Testelementen, die keine Verzögerungszone enthalten, länger in der Reagenzienzone gehalten. Es ist ebenfalls möglich, eine Verzögerungszone zwischen Reagenzienzone und Abtrennzone zu schaffen, indem ein poröses Matrixmaterial, beispielsweise ein Vlies, mit einem niedrigeren Querschnitt verglichen mit dem Querschnitt der angrenzenden Zonen als Verbindungszone bzw.Transportzone montiert wird. Auf diese Weise wird ein Probenflüssigkeitstransportengpass kreiert, der ebenfalls dazu führt, daß die Probenflüssigkeit länger in der Reagenzienzone verweilt, als dies der Fall wäre, wenn keine solche Verzögerungszone vorhanden wäre.

Ist der geschwindigkeitsbestimmende Schritt nicht im Lösen der Reagenzien zu sehen sondern in der Komplexbildung zwischen glykiertem Hämoglobin und den signalbildenden Molekülen, so ist es ausreichend, als Verzögerungszone eine lange, poröse Transportstrecke oder ein Reservoir zur Aufnahme der Probenflüssigkeit, die gelöste Reagenzien aus der Reagenzienzone enthält, beispielsweise in Form eines voluminösen Vlieses mit hoher Speicherkapazität für Flüssigkeit, zwischen Reagenzienzone und Abtrennzone vorzusehen.

Die Abtrennzone dient der Separation von in der Probenflüssigkeit gelösten, signalbildenden Molekülen von der Probenflüssigkeit, insbesondere von Hämoglobin und den in der Reagenzienzone gebildeten Komplexen aus glykiertem Hämoglobin und signalbildenden Molekülen. Vorzugsweise enthält die Abtrennzone eine poröse Membran oder ein Vlies, die jeweils durch die Probenflüssigkeit benetzbar sind.

Die Abtrennwirkung der Abtrennzone für die signalbildenden Moleküle kann auf den oben im Zusammenhang mit dem erfindungsgemäßen Verfahren beschriebenen Prinzipien beruhen. Wird fiir das Abtrennen ausgenutzt, daß die signalbildenden Moleküle einen Antikörper gegen glykiertes Hämoglobin tragen, so ist in der Abtrennzone des erfindungsgemäßen Testelements vorzugsweise ein immobilisiertes Antigen oder Hapten bereitgestellt, beispielsweise kovalent immobilisiert an Fasern eines Vlieses.

Die Abtrennung kann ebenfalls darauf beruhen, daß die signalbildenden Moleküle, vorzugsweise die oben beschriebenen Boronsäure-Farbstoff-Konjugate, bei pH-Werten, die mindestens um eine Einheit größer sind als der pKₛ der Boronsäure-Farbstoff-Konjugate, die freien, das heißt nicht an glykiertes Hämoglobin gebundenen Boronsäure-Farbstoff-Konjugate, verglichen mit Hämoglobin sowie mit dem Komplex aus glykiertem Hämoglobin und Boronsäure-Farbstoff-Konjugat eine höhere negative Ladungsdichte aufweist. Das Abtrennen der freien Boronsäure-Farbstoff-Konjugate von der Probe kann in diesem Fall beispielsweise dadurch erzielt werden, daß die Probe durch ein poröses, durchströmungsfähiges Material geleitet wird, welches nach außen hin positiv geladen ist bzw. positiv geladene Gruppen enthält. Beispielsweise haben sich hierfür Membranen, insbesondere solche aus Nylon als geeignet erwiesen, wobei bevorzugt ein mit quartären Ammoniumgruppen modifiziertes Nylon 66™, welches beispielsweise als Biodyne B™ von der Fa. Pall vertrieben wird, geeignet ist. Während die Probe und insbesondere das darin enthaltene Hämoglobin sowie der Komplex aus glykiertem Hämoglobin und Boronsäure-Farbstoff-Konjugat schnell in und durch das poröse, durchströmungsfähige Material strömen kann, ist dies aufgrund elektrostatischer Wechselwirkungen mit dem porösen Matrixmaterial dieser Zone für die freien Boronsäure-Farbstoff-Konjugate nicht möglich.

In all den genannten Fällen ist darauf zu achten, daß die Menge der zur Abtrennung der signalbildenden Moleküle befähigten Agentien (d. h. immobilisierte Bindungspartner, oberflächlich vorhandene Gruppen oder Ladungen) ausreichend bemessen ist, um den nicht an glykiertes Hämoglobin gebundenen Anteil der signalbildenden Moleküle zuverlässig aus der Probe zu entfernen, damit in der Nachweiszone nur diejenigen signalbildenden Moleküle ankommen, die an glykiertes Hämoglobin gebunden sind. Zusätzlich ist zu beachten, daß durch die abtrennenden Agentien nicht die Komplexe aus glykiertem Hämoglobin und signalbildenden Molekülen zerstört werden. Erfindungsgemäß ist es deshalb besonders bevorzugt, die Abtrennung von nicht-gebundenen signalbildenden Molekülen durch den Einsatz von positiv geladenen Membranen zu erreichen. Insbesondere haben sich hierfür die bereits erwähnten Membranen aus Nylon als geeignet erwiesen.

Bei dieser bevorzugten Ausführungsform des erfindungsgemäßen Testelements ist es fiir eine optimale Abtrennwirkung einerseits und eine optimale Stabilität des Komplexes aus glykiertem Hämoglobin und signalbildendem Molekül (d. h. Boronsäure-Farbstoff-Konjugat) andererseits wichtig, daß in der Probenflüssigkeit während der Analyse wohldefinierte Bedingungen herrschen. Insbesondere ist auf den pH-Wert zu achten. Durch diesen ist zum einen die Komplexbildung zwischen glykiertem Hämoglobin und Boronsäure-Farbstoff-Konjugat zu beeinflussen und zum anderen die Wanderungsgeschwindigkeit des freien Konjugats durch die Membran bestimmt. Bevorzugt ist es, daß der pH-Wert der Probenlösung ca. eine Größenordung größer ist als der pKₛ- Wert des Boronsäure-Farbstoff- Konjugats. Beispielsweise hat sich bei einem pKₛ- Wert des Boronsäure-Farbstoff- Konjugats von 7,5 ein pH-Wert von 8,5 als besonders geeignet herausgestellt. Erfindungsgemäß hat es sich als vorteilhaft herausgestellt, den pH-Wert durch Puffersubstanzen in der Reagenzienzone einzustellen. Vorteilhaft hat sich hierfür insbesondere Cyclohexylaminopropysulfonsäure (CAPS) erwiesen. Dem Fachmann ist natürlich geläufig, daß im Zusammenhang mit biologischen Proben nicht beliebige pH-Werte eingestellt werden können, ohne die Probe unbrauchbar zu machen oder gegebenenfalls weitere, beispielsweise zur Detektion eines Enzymlabels erforderliche Reaktionen ungünstig zu beeinflussen. Gegebenenfalls kann es deshalb erforderlich sein, den pKₛ-Wert des Boronsäure-Farbstoffkonjugats durch geeignete Substitution zu beeinflussen, um nicht in ungünstig hohen oder niedrigen pH-Wertbereichen arbeiten zu müssen. Auch solche Maßnahmen sind dem Fachmann an sich bekannt.

An die Abtrennzone schließt sich direkt oder indirekt eine in Transportrichtung der Probenflüssigkeit stromabwärts gelegene Nachweiszone an. In ihr können gegebenenfalls Nachweisreagenzien für die signalbildenden Molekülreste der signalbildenenden Moleküle, beispielsweise farbgebende Enzymsubstrate bei der Verwendung von Enzymlabeln, vorhanden sein. Vorzugsweise ist die Nachweiszone Bestandteil des porösen Materials, das die Abtrennzone enthält, der jedoch räumlich von der Abtrennzone getrennt ist.

Das erfindungsgemäße Verfahren bzw. das Erfindungsgemäße Testelement weist folgende Vorteile auf:
◆ Die aufwendige Abtrennung der Hämoglobine von der Probe oder gar die Auftrennung der Hämoglobine in einzelne Fraktionen wird vermieden. Stattdessen werden die signalbildenden Moleküle nach der Komplexbildung mit glykiertem Hämoglobin abgetrennt, was technisch einfacher zu realisieren ist und sogar in einem Durchflußverfahren, beispielsweise auf Testelementen, durchgeführt werden kann. Vorbehandlung der Probe und aufwendige Zwischenschritte entfallen. Das Verfahren ist dadurch auch von ungeschulten Personen durchzuführen, beispielsweise mit entsprechenden Testelementen von Diabetikern. Die Meßergebnisse stehen schnell, typischerweise innerhalb weniger Minuten, zur Verfügung.
◆ Zur Durchführung des Verfahren auf Testelementen ist in einer bevorzugten Ausführungsform lediglich die Probenflüssigkeit aufzugeben und in der Nachweiszone die Signale fiir Hämoglobin und glykiertes Hämoglobin zu bestimmen. Weitere Flüssigkeiten sind nicht zu dosieren.
◆ Bereits geringe Probenvolumina reichen zur Durchführung des Verfahren aus. Bevorzugte Ausführungsformen des erfindungsgemäßen Testelements benötigen nur wenige Mikroliter Blut, so daß die Probennahme beim Patienten schmerzarm, auf jeden Fall ohne Venenpunktion durchzuführen ist.
◆ Die Ergebnisse der Bestimmung des Verhältnisses von glykiertem zu nicht glykiertem bzw. zu Gesamthämoglobin sind bei Durchführung des erfindungsgemäßen Verfahrens mit dem erfindungsgemäßen Testelement in weiten Bereichen volumenunabhängig.

Die Erfindung wird durch die nachfolgenden Beispiele und Zeichnungen näher erläutert.

Figur 1 und Figur 2 zeigen schematisch jeweils einen seitlichen Schnitt durch zwei bevorzugte Ausführungsformen des erfindungsgemäßen Testelements, bei dem die Transportrichtung der Probenflüssigkeit im wesentlichen parallel zur Längsachse des Testelements ist.

Figur 3 zeigt schamatisch einen seitlichen Schnitt durch eine weitere bevorzugte Ausführungsform des erfindungsgemäßen Testelements, bei dem die Transportrichtung der Probenflüssigkeit im wesentlichen senkrecht zur Längsachse des Testelements ist.

Figur 4 zeigt schamatisch einen seitlichen Schnitt durch eine weitere bevorzugte Ausführungsform des erfindungsgemäßen Testelements.

Figur 5 zeigt eine durch lineare Regression erhaltene Kalibrationsgerade für Diabetikerblute, bei welcher der Kubelka-Munk-Quotient (KM, y-Achse) gegen den relativen Anteil an HbAlc bzogen auf Gesamthämoglobin (% HbAlc, x-Achse) aufgetragen wurde.

Die Ziffern in den Figuren bedeuten:
1 Träger
2 Gewebe
3 erstes poröses Matrixmaterial (Vlies)
4 zweites poröses Matrixmaterial (Membran)
5 Klebstoff
6 Probenaufgabezone
7 Reagenzienzone
8 Abtrennzone
9 Nachweiszone
10 Verzögerungszone
11 Detektionsöffnung
12 Probenaufgabeöffnung
13 Gehäusehälfte

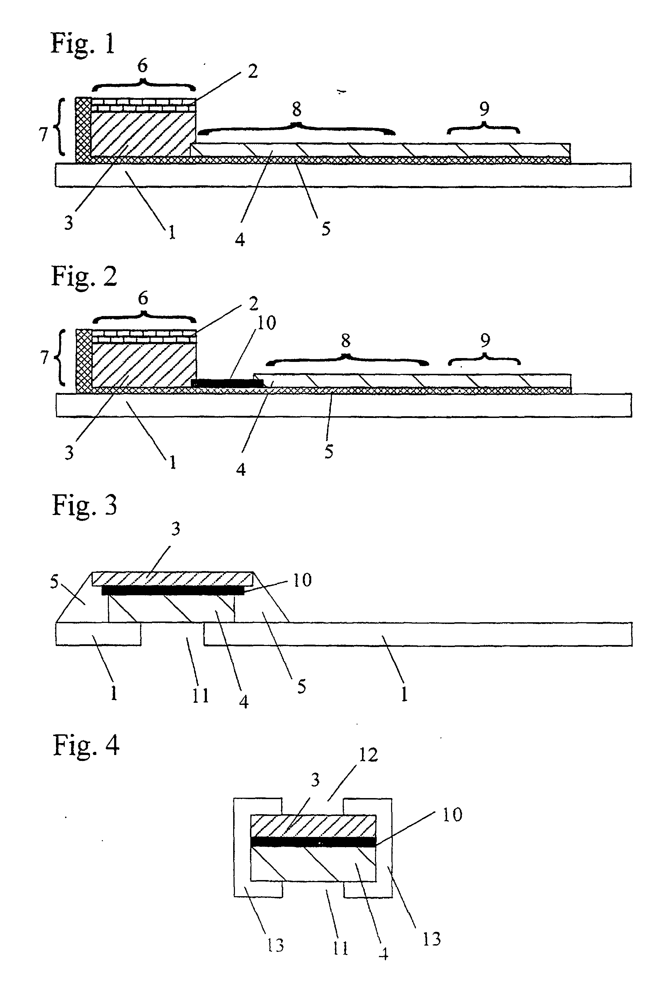

In Figur 1 ist ein schematischer Längsschnitt durch eine bevorzugte, streifenförmige Ausführungsform des erfindungsgemäßen Testelements abgebildet. Auf einem streifenförmigen Träger (1) sind mit Hilfe eines Klebstoffs (5), beispielsweise eines Schmelzklebers oder eines doppelseitigen Klebebandes, ein Gewebe (2), ein erstes poröses Matrixmaterial (Vlies) (3) sowie ein zweites poröses Matrixmaterial (Membran) (4) montiert. Das Gewebe (2) überdeckt das erste poröse Matrixmaterial (3) und das erste poröse Matrixmaterial (3) überlappt geringfügig mit dem zweiten porösen Matrixmaterial (4), um so einen Flüssigkeitsübertritt zwischen den Materialien zu ermöglichen.

Die dem Träger (1) abgewandte Oberfläche des Gewebes (2) dient als Probenaufgabezone (6). Im Gewebe (2) und im ersten porösen Matrixmaterial (3) sind die Reagenzien enthalten, die für die selektive Markierung des glykierten Hämoglobins in der Probe erforderlich sind, beispielsweise Puffersalze, Hämolysereagenzien und signalbildende Moleküle. Das Gewebe (2) und das erste poröse Matrixmaterial (3) dienen somit als Reagenzienzone (7).

Derjenige Abschnitt des zweiten porösen Matrixmaterials (4), welcher der Reagenzienzone (7) benachbart ist, dient dazu, freie signalbildende Moleküle, die durch die Probenflüssigkeit aus der Reagenzienzone (7) gelöst wurden und nicht mit glykiertem Hämoglobin reagieren konnten, aus der Probenflüssigkeit abzufangen. Dieser Bereich wird als Abtrennzone (8) bezeichnet. Je nach Prinzip der Abtrennung können in der Abtrennzone (8) immobilisiert selektive Bindungspartner für die signalbildenden Moleküle vorliegen oder aber das Matrixmaterial (4) selbst kann die Abtrennung, beispielsweise aufgrund elektrostatischer Wechselwirkungen mit den in der Probe gelösten, freien signalbildenden Molekülen, bewirken.

An die Abtrennzone (8) im zweiten porösen Matrixmaterial (4) schließt sich die Nachweiszone (9) an, in der Gesamthämoglobin und das mit signalbildenden Molekülen markierte glykierte Hämoglobin detektiert werden. Dies kann bspw. entweder direkt durch Messung der Reflektion von Hämoglobin und entsprechender Eigenschaften der signalbildenden, an glykiertes Hämoglobin gebundenen Markierung (bspw. Direktlabel, Radiolabel) geschehen oder indirekt über Reaktionsprodukte, die sich unter Einwirken der signalbildenden Markierung bilden (bspw. Enzymlabel). Im letztgenannten Fall kann es erforderlich sein, daß in der Nachweiszone (9) Nachweisreagenzien zur Verfügung gestellt werden, die in Gegenwart der signalbildenden Markierung ein detektierbares Signal erzeugen können. Die Detektion kann hier entweder von derjenigen Seite des zweiten porösen Matrixmaterials (4) erfolgen, die dem Träger (1) abgewandt ist. In diesem Fall ist es möglich, den Träger (1) aus einem nichttransparenten Material zu fertigen. Es ist jedoch auch möglich, die Detektion von der dem Träger (1) zugewandten Seite durchzuführen. Dies ist beispielsweise in einer bevorzugten Ausführungsform möglich, bei der transparente Materialien für Träger (1) und Klebstoff (5) verwendet werden oder bei der die Detektion durch eine Öffnung im Träger (1) und der Klebstoffschicht (5) im Bereich der Nachweiszone (9) erfolgt.

Das in Figur 2 in einem schematischen Längsschnitt abgebildete streifenförmige Testelement entspricht im wesentlichen dem Testelementeaufbau, wie er im Zusammenhang mit Figur 1 beschrieben ist. Im Unterschied zu dem Testelement aus Figur 1 befindet sich in dieser weiteren bevorzugten Ausführungsform zwischen der Reagenzienzone (7) und der Abtrennzone (8), das heißt, zwischen erstem porösem Matrixmaterial (3) und zweitem porösem Matrixmaterial (4) eine Verzögerungszone (10). In der in Figur 2 abgebildeten besonders bevorzugten Ausführungsform ist die Verzögerungszone (10) durch ein weiteres poröses Matrixmaterial realisiert, welches eine deutlich kleineren Querschnitt aufweist, als dies das erste poröse Matrixmaterial (3) und das zweite poröse Matrixmaterial (4) tun. Dadurch wird der Probenflüssigkeitstransport von der Reagenzienzone (7) in die Abtrennzone (8) im Vergleich zu der Ausführungsform, wie sie in Figur 1 dargestellt ist, verlangsamt. Die Probenflüssigkeit befindet sich durch diese Maßnahme länger in der Reagenzienzone (7), wo die längere Verweilzeit dazu genutzt wird, die Reagenzien aus der Reagenzienzone (7) zu lösen und die Komplexbildung zwischen signalbildenden Molekülen und glykiertem Hämoglobin zu erlauben.

Während der Probenflüssigkeitstransport in den streifenförmigen Testelementen der Figuren 1 und 2 im wesentlichen parallel zur Längsachse des Testelements stattfindet, ist in Figur 3 eine weitere bevorzugte Ausführungsform eines streifenförmigen Testelemtents im schematischen Längsschnitt abgebildet, bei dem der Probenflüssigkeitstransport im wesentlichen senkrecht zur Längsachse des Testelements stattfindet. In dem hier abgebildeten bevorzugten Testelement wird Probenflüssigkeit auf die Oberseite, das heißt, die dem Träger (1) abgewandte Seite des ersten porösen Matrixmaterials (3) aufgegeben. Die Detektion von Hämoglobin und glykiertem Hämoglobin erfolgt in diesem Fall durch eine Öffnung (11) im Träger (1), die beispielsweise als kreisrundes Loch oder als rechteckiger Schlitz gestaltet sein kann.

Während in den Figuren 1 und 2 die einzelnen funktionalen Zonen des erfindungsgemäßen Testelements im wesentlichen nebeneinander auf dem Träger (1) angeordnet sind, sind diese Zonen in der Ausführungsform, wie sie in Figur 3 dargestellt ist, im wesentlichen übereinander, quasi als stapelartiger Aufbau, angebracht. Das zweite poröse Matrixmaterial (4), die Verzögerungszone (10) und das erste poröse Matrixmaterial (3) sind - in dieser Reihenfolge - schichtweise auf dem Träger (1) befestigt, wozu sich insbesondere Schmelzkleber (5) eignet, der die einzelnen Schichten von deren Rand her miteinander in Kontakt hält und auf dem Träger (1) befestigt.

Da das Abtrennen der nicht an glykiertes Hämoglobin gebundenen signalbildenden Moleküle direkt über der Detektionsöffnung (11) des erfindungsgemäßen Testelements im zweiten porösen Matrixmaterial (4) stattfindet und sich deswegen die nicht-gebundenen signalbildenden Moleküle direkt oberhalb der Detektionsöffnung (11) innerhalb des zweiten porösen Matrixmaterials (4) befinden, ist es vorteilhaft, daß das zweite poröse Matrixmaterial (4) optisch undurchsichtig ist, so daß nur Probenmaterial beobachtbar ist, das an derjenigen Oberfläche des zweiten porösen Matrixmaterials (4) angelangt, welche durch die Detektionsöffnung (11) freigelassen ist.

Die Verzögerungszone (10) ist im Fall einer übereinanderliegenden Anordnung der einzelnen Zonen (6-10), wie sie in Figur 3 und Figur 4 gezeigt ist, vorzugsweise als Schicht einer langsam löslichen Substanz auf das erste (3) oder zweite (4) poröse Matrixmaterial aufgebracht. Alternativ kann sie auch als hydrophobierende Beschichtung auf einem der beiden Matrixmaterialien (3, 4) ausgestaltet sein.

In Figur 4 ist eine weitere bevorzugte Ausführungsform des erfindungsgemäßen Testelements abgebildet. In Analogie zu Figur 3 sind das erste poröse Matrixmaterial (3), die Verzögerungszone (10) und das zweite poröse Matrixmaterial (4) stapelartig übereinander geschichtet. Während in Figur 3 dieser stapelartige Verbund auf einem streifenförmigen Trägermaterial (1) angeordnet ist, zeichnet sich die Ausführungsform der Figur 4 dadurch aus, daß der stapelartige Schichtenaufbau durch zwei Gehäusehälften (13) zusammengehalten wird. Das Gehäuse, sowie die darin zusammengehaltenen Schichten können dabei jeden beliebigen Grundriß aufweisen, beispielsweise rund, quadratisch oder rechteckig sein. Zur Probenaufgabe ist eine Probenaufgabeöffnung (12) vorgesehen. Die Detektion überfolgt über eine Detektionsöffnung (11). Beide Öffnungen (11, 12) können sich direkt gegenüberliegen oder aber versetzt angeordnet sein.

### Beispiel 1: Herstellung des erfindungsgemäßen Testelements gemäß Figur 1

Ein 6 mm breites und 11 mm langes Stück einer positiv geladenen, 150 µm mm dicken Nylon-Membran (Biodyne B™, 3 µm Porenweite, Pall) wurde mit Hilfe eines doppelseitigen Klebebandes auf einen 6 mm breiten und 50 mm langen Streifen einer transparenten Trägerfolie aus Polyester (Melinex O klar™, Dicke 175 µm, ICI) geklebt.

Ein 6 mm breites, 4,5 mm langes und 720 µm hohes Stück Glasfaservlies (60 g/m²) wurde mit einer Lösung enthaltend Cyclohexylaminopropylsulfonsäure (CAPS) als Puffersubstanz und dem gemäß US 5,631,364 erhältlichen Boronsäure-Farbstoff-Konjugat XC-DAPOL-CPBA (5-[[[3-[(4-Boronobenzoyl)-amino]-2-hydroxypropyl]amino]sulfonyl]-2-[[4gethylarnino)-3-methylphenyl] [4-(ethylimino)-3-methyl-2,5-cyclohexadien-1-yliden]-methyl]-benzolsulfonsäure, CAS-Nr. 191231-97-7, Formel II) getränkt und getrocknet.

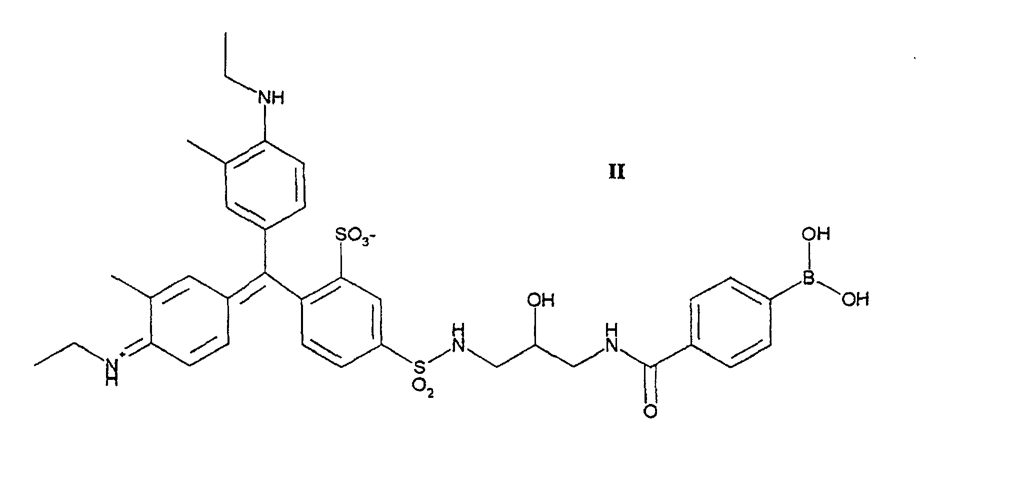

Das derart präparierte, getrocknete Vlies enthielt:

| | |
|---|---|
| Boronsäure-Farbstoff-Konjugat | 10 µg |
| CAPS | 0,44 mg |

Ein 6 mm breites und 5 mm langes Polyestergewebe (280 HC, Dicke 250 µm, Sefar) wurde mit einer Lösung enthaltend Saponin, Dioctylnatriumsulfonat und Diamylnatriumsulfonat getränkt und getrocknet. Das Gewebe enthielt:

| | |
|---|---|
| Saponin | 60 µg |
| Dioctylnatriumsulfonat | 15 µg |
| Diamylnatriumsulfonat | 15 µg |

Das derartig behandelte Gewebe wurde mit Hilfe eines doppelseitigen Klebebandes auf das zuvor ebenfalls mit doppelseitigem Klebeband auf der Trägerfolie befestigten Vlies montiert, so daß das Gewebe das Vlies ganz überdeckte und zwischen Vlies und Mambran eine schmale Überlappung von ca. 1 mm zustande kam. Im fertigen, streifenförmigen Testelement übernahm das Gewebe die Funktion der Probenaufgabezone, und Gewebe und Glasfaservlies die Funktion der Reagenzienzone. Der dem Vlies benachbarte Bereich der Membran diente als Abtrennzone, während der dem Vlies abgewandte Bereich der Mambran als Nachweiszone diente.

### Beispiel 2: Bestimmung des relativen Anteils von glykiertem Hämoglobin am Gesamthämoglobin mit Hilfe des erfindungsgemäßen Verfahrens unter Verwendung des Testelements aus Beispiel 1

Auf die frei liegende Oberfläche des Gewebes des Testelements aus Beispiel 1 wurde Blut als Probenmaterial aufgegeben. Nach Erreichen der Detektionszone durch das Probenmaterial wurden bei Wellenlängen von 540 nm für die Bestimmung von Gesamthämoglobin, bei 630 nm für die Bestimmung von glykiertem Hämoglobin und bei 700 nm für die Bestimmung der Transparenz des Testelements in der Detektionszone jeweils die Remissionswerte ermittelt. Die Konzentration von Gesamthämoglobin und glykiertem Hämoglobin wurden nach der Kubelka-Munk-Gleichung berechnet und nach Division mit einem Umrechnungsfaktor multipliziert, um den relativen Anteil an HbAlc zu erhalten.

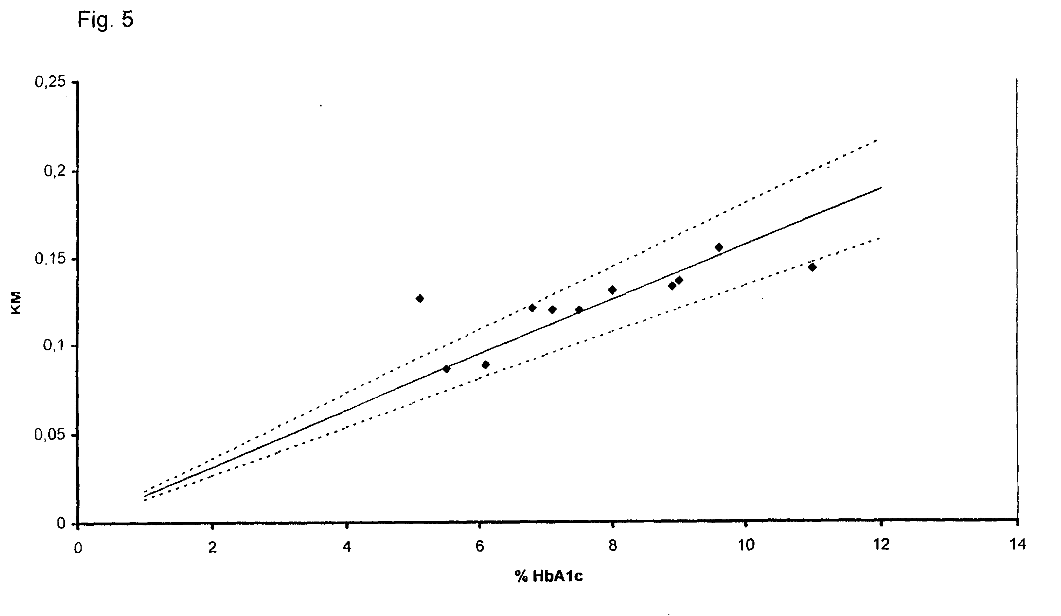

In Figur 5 sind die Ergebnisse einer Meßreihe mit unterschiedlichen Diabetikerbluten dargestellt.

## Patentansprüche

1. Verfahren zur Bestimmung des Verhältnisses von glykiertem zu nichtglykiertem Hämoglobin in einer Probe, wobei man
(i) gegebenenfalls die Probe hämolysiert, um zellgebundenes Hämoglobin freizusetzen,
(ii) die Probe mit einem Überschuß an signalbildenden Molekülen in Kontakt bringt, wobei ein Teil der signalbildenden Moleküle an glykiertes Hämoglobin in der Probe bindet,
(iii) den nicht an glykiertes Hämoglobin gebundenen Teil der signalbildenden Moleküle von der Probe abtrennt,
(iv) in der Probe, die an glykiertes Hämoglobin gebundene signalbildende Moleküle sowie nicht-glykiertes Hämoglobin enthält, die signalbildenden Moleküle als Maß für das glykierte Hämoglobin und die Gesamtmenge an Hämoglobin bestimmt, und
(v) aus den gemäß Schritt (iv) erhaltenen Werten das Verhältnis von glykiertem zu nicht-glykiertem Hämoglobin in der Probe bestimmt.

2. Verfahren gemäß Anspruch 1, **dadurch gekennzeichnet, daß** die signalbildenden Moleküle ein Konjugat aus einem oder mehreren Dihydroxyborylresten oder Salzen davon umfassen, die an eine signalbildende Markierung gebunden sind.

3. Verfahren gemäß Anspruch 2, **dadurch gekennzeichnet, daß** die signalbildenden Moleküle Boronsäurekonjugatverbindungen mit einem Absorptionsmaximum von nicht weniger als 600 nm oder deren Salze sind, wobei die Verbindungen die allgemeine Formel I besitzen,
**V-W-B(OH)**_{**2**} (I)
in der V ein Cyanin-, Phenoxazin-, Phenothiazin- oder Triphenylmethanchromophor- und/oder -fluorophorrest ist, der ein Absorptionsmaximum bei nicht weniger als 600 nm aufweist, und W eine organische Brückengruppe ist.

4. Verfahren gemäß einem der Ansprüche 1 bis 3, **dadurch gekennzeichnet, daß** der nicht an glykiertes Hämoglobin gebundene Teil der signalbildenden Moleküle über elektrostatische Wechselwirkungen abgetrennt wird.

5. Verfahren gemäß einem der Ansprüche 1 bis 4, **dadurch gekennzeichnet, daß** man in der Probe, die an glykiertes Hämoglobin gebundene signalbildende Moleküle sowie nichtglykiertes Hämoglobin enthält, die signalbildenden Moleküle als Maß für das glykierte Hämoglobin durch die Messung von Absorption, Reflektion oder Fluoreszenz bestimmt.

6. Analytisches Testelement, das zur Bestimmung des Verhältnisses von glykiertem zu nicht-glykiertem Hämoglobin in einer Probe anhand des Verfahrens gemäß Anspruch 1 geeignet ist, enthaltend neben- und/oder übereinander angeordnet
- eine Probenaufgabezone,
- eine Reagenzienzone, in der signalbildende Moleküle nicht-immobilisiert vorliegen und/oder die Hämolysereagenzien zur Freisetzung von zellgebundenem Hämoglobin enthält,
- eine Abtrennzone zum Abtrennen von nicht an glykiertem Hämoglobin gebundenen signalbildenden Molekülen, sowie
- eine Nachweiszone, wobei
die Zonen auf oder in einem oder mehreren porösen Matrixmaterialien untergebracht sind und untereinander in einem Flüssigkeitsübertritt ermöglichendem Kontakt stehen oder in einen solchen gebracht werden können.

7. Analytisches Testelement gemäß Anspruch 6, **dadurch gekennzeichnet, daß** die Abtrennzone ein poröses Matrixmaterial enthaltend positiv geladene Gruppen ist.

8. Analytisches Testelement gemäß Anspruch 6, **dadurch gekennzeichnet, daß** zwischen Reagenzienzone und Abtrennzone eine Verzögerungszone liegt.

9. Analytisches Testelement gemäß einem der Ansprüche 6 bis 8, **dadurch gekennzeichnet, daß** das oder die porösen Matrixmaterialien auf einem inerten Träger angebracht sind.

10. Verwendung eines analytischen Testelements gemäß einem der Ansprüche 6 bis 9 zur Bestimmung von glykiertem Hämoglobin in einer Probe.

## Claims

1. Method for the determination of the ratio of glycated to non-glycated haemoglobin in a sample wherein
(i) the sample is optionally haemolysed in order to release cell-bound haemoglobin,
(ii) the sample is contacted with an excess of signal-generating molecules during which a fraction of the signal-generating molecules binds to glycated haemoglobin in the sample,
(iii) the fraction of signal-generating molecules that is not bound to glycated haemoglobin is separated from the sample,
(iv) the signal-generating molecules are determined in the sample which contains signal-generating molecules bound to glycated haemoglobin as well as non-glycated haemoglobin as a measure for the glycated haemoglobin and the total amount of haemoglobin is determined, and
(v) the ratio of glycated to non-glycated haemoglobin in the sample is determined from the values obtained according to step (iv).

2. Method as claimed in claim 1, wherein the signal-generating molecules comprise a conjugate of one or several dihydroxyboryl residues or salts thereof which are bound to a signal-generating label.

3. Method as claimed in claim 2, wherein the signal-generating molecules are boronic acid conjugate compounds with an absorption maximum of not less than 600 nm or salts thereof wherein the compounds have the general formula I
**V-W-B(OH)2** (I)
in which V is a cyanine, phenoxazine, phenothiazine or triphenylmethane chromophore and/or fluorophore residue which has an absorption maximum of not less than 600 nm and W is an organic bridge group.

4. Method as claimed in one of the claims 1 to 3, wherein the fraction of the signal-generating molecules that is not bound to haemoglobin is separated by electrostatic interactions.

5. Method as claimed in one of the claims 1 to 4, wherein the signal-generating molecules are determined in the sample which contains signal-generating molecules bound to glycated haemoglobin as well as non-glycated haemoglobin as a measure for glycated haemoglobin by measuring absorbance, reflection or fluorescence.

6. Analytical test element which is suitable for determining the ratio of glycated to non-glycated haemoglobin in a sample by a method as claimed in claim 1 which contains next to and/or above one another
- a sample application zone
- a reagent zone in which non-immobilized signal-generating molecules are present and/or which contains the haemolysis reagents to release cell-bound haemoglobin,
- a separation zone for separating signal-generating molecules that are not bound to glycated haemoglobin and
- a detection zone, wherein
the zones are located on or in one or several porous matrix materials and are in contact with one another enabling liquid transfer or can be brought into such a contact.

7. Analytical test element as claimed in claim 6, wherein the separation zone is a porous matrix material containing positively charged groups.

8. Analytical test element as claimed in claim 6, wherein a retardation zone is located between the reagent zone and separation zone.

9. Analytical test element as claimed in one of the claims 6 to 8, wherein the porous matrix material or materials are mounted on an inert support.

10. Use of an analytical test element as claimed in one of the claims 6 to 9 for the determination of glycated haemoglobin in a sample.

## Revendications

1. Procédé pour la détermination du rapport de l'hémoglobine glycosylée à l'hémoglobine non glycosylée dans un échantillon, dans lequel
(i) le cas échéant, on soumet l'échantillon à une hémolyse dans le but de libérer l'hémoglobine liée aux cellules ;
(ii) on met l'échantillon en contact avec un excès de molécules formant des signaux, une partie des molécules formant des signaux se liant à l'hémoglobine glycosylée dans l'échantillon ;
(iii) on sépare de l'échantillon la fraction des molécules formant des signaux, non liée à de l'hémoglobine glycosylée ;
(iv) dans l'échantillon, qui contient des molécules formant des signaux liées à de l'hémoglobine glycosylée, ainsi que de l'hémoglobine non glycosylée, on détermine les molécules formant des signaux comme mesure pour la teneur en hémoglobine glycosylée, ainsi que la quantité totale d'hémoglobine ; et
(v) à partir des valeurs obtenues conformément à l'étape (iv), on détermine le rapport de l'hémoglobine glycosylée à l'hémoglobine non glycosylée dans l'échantillon.

2. Procédé selon la revendication 1, **caractérisé en ce que** les molécules formant des signaux comprennent un conjugué d'un ou de plusieurs radicaux dihydroxyboryle ou de leurs sels qui sont liés à un marqueur formant des signaux.

3. Procédé selon la revendication 2, **caractérisé en ce que** les molécules formant des signaux sont des composés de conjugués d'acide borique possédant un maximum d'absorption qui n'est pas inférieur à 600 nm ou de leurs sels, les composés répondant à la formule générale I
V-W-B(OH)₂
dans laquelle V représente un radical de chromophore ou de fluorophore à base de cyanine, de phénoxazine, de phénothiazine ou de triphénylméthane, qui présente un maximum d'absorption qui n'est pas inférieur à 600 nm, et W représente un groupe organique faisant pont.

4. Procédé selon l'une quelconque des revendications 1 à 3, **caractérisé en ce que** la fraction des molécules formant des signaux, non liée à l'hémoglobine glycosylée est séparée par des interactions électrostatiques.

5. Procédé selon l'une quelconque des revendications 1 à 4, **caractérisé en ce qu'**on détermine, dans l'échantillon, qui contient des molécules formant des signaux liées à de l'hémoglobine glycosylée, ainsi que de l'hémoglobine non glycosylée, les molécules formant des signaux comme mesure pour la teneur en hémoglobine glycosylée, en mesurant l'absorption, la réflexion ou la fluorescence.

6. Élément d'essai analytique qui est approprié pour la détermination du rapport de l'hémoglobine glycosylée à l'hémoglobine non glycosylée dans un échantillon conformément au procédé selon la revendication 1, contenant, à l'état disposé l'une à côté de l'autre et/ou l'une par-dessus l'autre,
- une zone de réception de l'échantillon,
- une zone réservée aux réactifs, dans laquelle sont présentes, à l'état non immobilisé, des molécules formant des signaux et/ou qui contient les réactifs d'hémolyse pour la libération de l'hémoglobine liée à des cellules,
- une zone de séparation pour la séparation des molécules formant des signaux non liées à de l'hémoglobine glycosylée, et
- une zone de décèlement, dans lequel
les zones sont appliquées sur ou dans une ou plusieurs matières matricielles poreuses et entrent dans un contact réciproque permettant le passage d'un liquide ou bien peuvent être amenées dans un tel contact.

7. Élément d'essai analytique selon la revendication 6, **caractérisé en ce que** la zone de séparation est une matière matricielle poreuse contenant des groupes chargés positivement.

8. Élément d'essai analytique selon la revendication 6, **caractérisé en ce qu'**une zone de ralentissement est disposée entre la zone réservée aux réactifs et la zone de séparation.

9. Élément d'essai analytique selon l'une quelconque des revendications 6 8, **caractérisé en ce que** la ou les matières matricielles poreuses sont appliquées sur un support inerte.

10. Utilisation d'un élément d'essai analytique selon l'une quelconque des revendications 6 à 9, pour la détermination d'hémoglobine glycosylée dans un échantillon.
